# EUROPEAN PATENT APPLICATION

(11) **EP 4 646 931 A1**
(43) Date of publication of application: **12.11.2025**
(21) Application number: 23882032.8
(22) Date of filing: 23.10.2023
(51) Int. Cl.: A01N 43/40, A01N 43/16, C07H 5/04, C07D 211/06, A01P 7/02, A01P 7/04

(54) **BIODEGRADABLE IMINOSUGARS AND AMINO CYCLITOL GLYCOSIDES INHIBITING ALPHA-GLUCOSIDASE WITH SYNERGISTIC INSECTICIDE OR ACARICIDE ACTIVITY OR BOTH**

(30) Priority: 24.10.2022 ES 202230916
(71) Applicant: Francisco Aragon S.L.U, 30500 Molina de Segura (Murcia) (ES)
(72) Inventor: CASAÑA GINER, Victor, 30500 Molina de Segura (Murcia) (ES); CHAFER DOLZ, Beatriz, 46011 Valencia (ES); VALVERDE ALMAIDA, Alicia, 30007 Murcia (ES); LÓPEZ CAPEL, Antonio, 30509 Llano de Molina (Murcia) (ES); MORENO FRESNEDA, Ana Rosa, 30100 Espinardo (Murcia) (ES); VILCHEZ JIMÉNEZ, Raquel, 30120 El Palmar (Murcia) (ES); NÚÑEZ DELICADO, Estrella, 30008 Murcia (ES); CECILIA CANALES, Jose Maria, 46011 Valencia (ES); IMBERNÓN TUDELA, Baldomero, 30850 Totana (Murcia) (ES); CERÓN CARRASCO, Jose Pedro, 30840 Murcia (ES)
(74) Representative: Soler Lerma, Santiago
(86) International application number: PCT/ES2023/070622
(87) International publication number: WO 2024/089310

(57) **Abstract**

The present invention relates to a new class of insecticides that can also be synergists of other insecticides and that comprise iminosugar and amino cyclitol glycoside compounds and/or alpha-glucosidase inhibitors, which are known for pharmaceutical use for diabetics and people with lysosomal diseases.

## Description

The invention relates to a new class of insecticides that may also be synergists of other insecticides. The insecticides object of the invention comprise iminosugar or amino cyclitol glycoside compounds and/or alpha-glucosidase inhibitors, known for pharmaceutical use for diabetics and people with lysosomal diseases. The activity thereof is relevant because it is relevant in insects of interest in Public Health and livestock such as cockroaches, mosquitoes and flies.

In the present disclosure, with the exception of the following paragraph, any reference to insecticides should also be understood as referring to acaricides, since known acaricides, with the exception of, for example, the use of formic acid, share practically all known mechanisms of insecticidal and synergistic action.

Insecticides may have an "insecticide" effect *per* se or an acaricide effect or both effects.

One of its main effects is the knockdown effect, which is the inability of an insect to walk normally although it may seem to be alive.

Throughout the disclosure, this effect will be referred to as *knockdown.*

The technical field to which they belong is that of biocidal products, which also includes synergists with biocides.

### BACKGROUND TO THE INVENTION

The present invention is encompassed within the field of insecticide and acaricide compounds, as well as in the synergistic compounds of insecticides and acaricides.

Iminosugars are known human alpha-glucosidase inhibitor compounds, and used for treatment of diabetes (by delaying glucose absorption) and other diseases related to carbohydrate metabolism.

In turn, certain compounds of the amino cyclitol glycoside type are used for the same purpose. Both types have also been described as alpha-amylase inhibitors, making it difficult to establish a dividing line of activity according to structure.

Both types of compounds (iminosugars and amino cyclitol glycosides) are also known for their influence on the development of certain insect species, with delayed effects interfering with development, but not as traditional, immediate-acting insecticides (understood as the death of adult individuals in less than 24 hours), as is the case of 1-deoxynojirimycin in Samia cynthia ricini ("A toxicological, metabonomic and transcriptional analysis to investigate the property of mulberry 1-deoxynojirimycin against the growth of Samia cynthia ricini"; (2018), Peng-Cheng et al., doi: 10.1016/j.pestbp.2018.08.009.

Both in agriculture and in urban pest control, as well as in homes, there is a pressing need to find new insecticides and also synergists, which enable avoiding the problem of resistance to current insecticides, and which allow the least use (in the case of synergy) of the insecticidal active compounds so that the toxicological and environmental burden of each molecule is reduced.

On the other hand, the world faces viral, bacterial and parasitic diseases by other microorganisms, transmitted by insects and mites, notably mosquitoes and to a lesser extent, lice, fleas and ticks, for which any progress in insect and mite control technology is relevant.

### DESCRIPTION OF THE INVENTION

The present invention presents a mode of action, hitherto not described, of molecules with biological effects on insects and mites, opening the way in the field of new modes of action and types of insecticides and/or synergists.

In particular, the aforementioned alpha-glucosidase inhibitors have some mild insecticidal activity and a synergistic effect that has been very high in our tests. Furthermore, these compounds have a low toxicological profile already studied in pharmacological applications, as well as suitability with respect to their effect on the environment. As sugarbased compounds, they enjoy remarkable biodegradation, which can bring benefits at human and environmental levels.

Object of this invention is an insecticide and/or acaricide formulation characterised in that it contains an alpha-glucosidase inhibitory molecule as an active ingredient. The person skilled in the art can obtain ways of formulating insecticides and/or acaricides in any reference manual, especially those contemplated in the "The Pesticide Manual", published by the British Crop Protection Council, in any of its versions to date.

### BRIEF DESCRIPTION OF THE FIGURES

For a better understanding of the invention and its behaviour, the following Figures are attached.

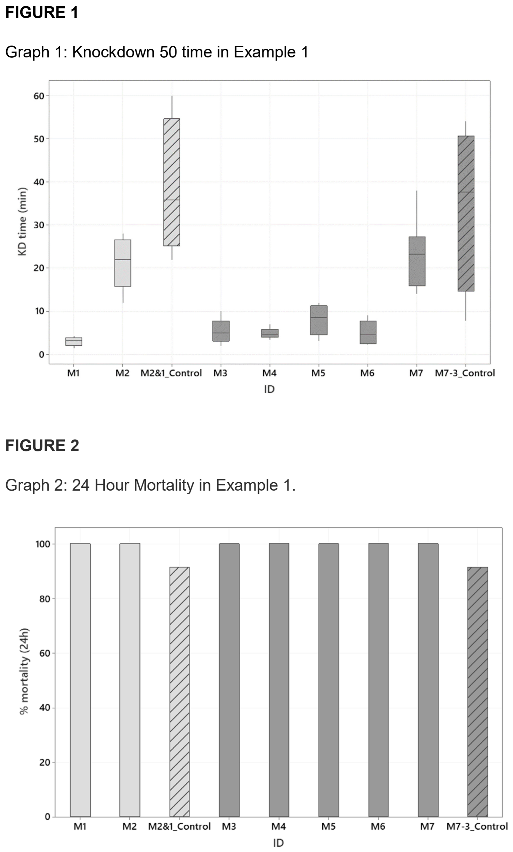

FIGURE 1 shows an explanatory graph of Knockdown 50 time in Example 1.
FIGURE 2 shows an explanatory graph of the 24-hour mortality in Example 1.

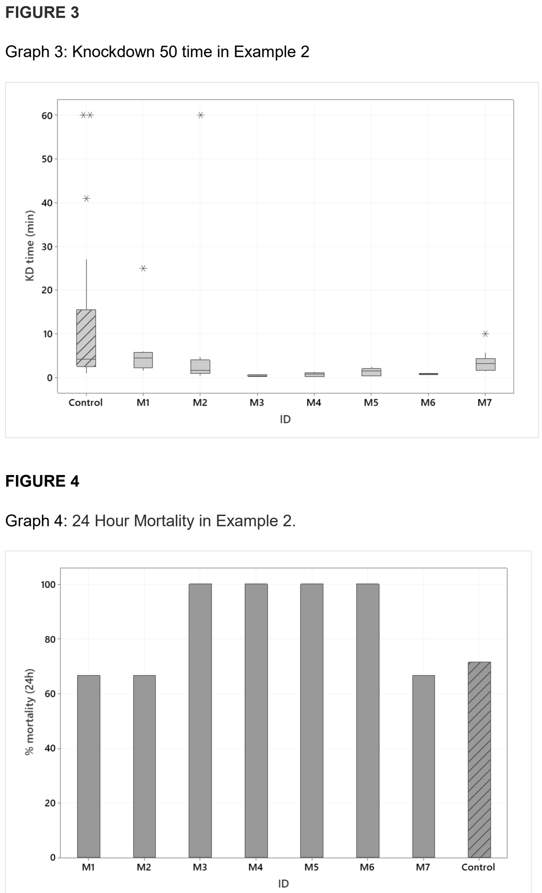

FIGURE 3 shows an explanatory graph of Knockdown 50 time in Example 2.
FIGURE 4 shows an explanatory graph of the 24-hour mortality in Example 2.

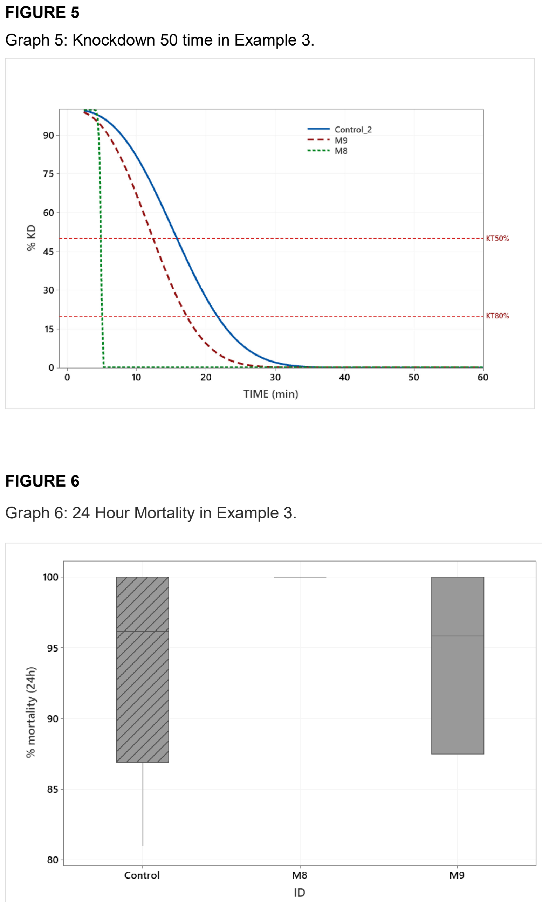

FIGURE 5 shows an explanatory graph of Knockdown 50 time in Example 3.

### DESCRIPTION OF AN EMBODIMENT

As stated, the subject of this invention is an insecticidal formulation characterised in that as it contains an alpha-glucosidase inhibitory molecule an active ingredient.

In more detail, the object of this invention is the use as insecticides and acaricides of the compounds (or molecules, used synonymously) characterised in that the structure corresponds to structure (I) or (II): 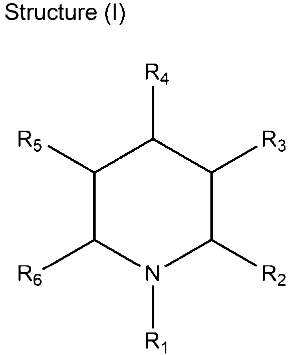 wherein,
R1 is chosen from: methyl, ethyl, propyl, butyl, pentyl, hexyl, heptyl, octyl, nonyl, decyl, undecanyl, dodecyl, tridecyl, tetradecyl, pentadecyl, hexadecyl, septadecyl, octadecyl; all of them alkanes comprising the linear chains and their positional isomers; hydroxymethyl, hydroxyethyl, hydroxypropyl, hydroxybutyl, hydroxypentyl, hydroxyhexyl, hydroxyheptyl, hydroxyoctyl, hydroxynonyl, hydroxydecyl, hydroxyundecanyl, hydroxydodecyl, hydroxytridecyl, hydroxytetradecyl, hydroxypentadecyl, hydroxyhexadecyl, hydroxyseptadecyl, hydroxyoctadecyl; all of them hydroxylated alkanes comprising the linear chains and their positional isomers which are hydroxylated, with the alcohol group being primary or secondary; heptan-4-yloxyhexyl, 5-butoxypentyl, 4,4,4-trifluorobutyl, 6-cyclohexyloxyhexyl, cyclopropylmethyl, dimethylpropylsilyl, cyclopentyloxyhexyl, trimethylsilanylmethyl, trimethylsilanylethyl, trimethylsilanylpropyl, trimethylsinanylbutyl, trimethylsilylpentyl, as well as the particular cases where R1 is dimethyl, diethyl, dipropyl, i.e. two alkyls bound to the nitrogen;
R2, R3, R4, R5 and R6, independently of each other are chosen from: hydrogen, hydroxyl, hydroxymethyl, hydroxyethyl, hydroxypropyl, hydroxybutyl;
so that molecule (I) is a diol, triol or tetrol, with the exception of hydroxyls that may exist in R1;
as well as their possible salts, including those of hydrochlorides, including that of 3,4,5-trihydroxy-2-(hydroxymethyl)piperidin-1-yl)heptane-1,2,3,4,5,6-hexol. 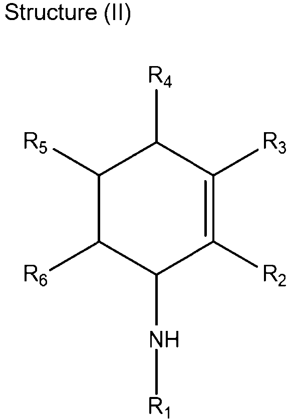
Wherein
R1 is chosen from: monosaccharide, disaccharide, trisaccharide, including isomaltotriose;
R2, R3, R4, R5 and R6, independently of each other are chosen from: hydrogen, hydroxyl, hydroxymethyl, hydroxyethyl, hydroxypropyl, hydroxybutyl;
or (II) is 5-(1,3-dihydroxypropan-2-ylamino)-1-(hydroxymethyl)cyclohexane-1,2,3,4-tetrol;
with (I) and (II) comprising any possible stereoisomeric form of the depicted structures.

From what has been observed, an insecticide and/or acaricide formulation with compounds of structure (I) and/or (II) with a Knockdown 50 effect at most in 24 hours is of interest, when the insect or mite is applied 50g of said molecule, directly or in an appropriate vehicle, per kg of weight.

And much more relevantly, it is of interest to use said compounds (I) and/or (II) in combination in an insecticide and/or acaricide formulation which further comprises an insecticide and/or acaricide of the state of the art, so that they act synergistically.

The object of this invention is also to combine compounds (I) and/or (II) with pyrethrins and/or pyrethroids.

Without being considered in a limiting way, the pyrethroid will preferably be chosen from prallethrin, 1R-trans-phenothrin, deltamethrin, tetramethrin, d-tetramethrin, metofluthrin, momfluorothrin, allethrin, cypermethrin, alpha-cypermethrin, beta-cypermethrin, zeta-cypermethrin, imiprothrin, lambda-cyhalothrin, gamma-cyhalothrin, bifenthrin, cyfluthrin, cyphenothrin, fenvalerate, fluvalinate, flumethrin, tralomethrin, resmethrin, kadethrin, permethrin, cis-permethrin, trans-permethrin, etofenprox, acrinathrin, flucythrinate, fenpropathrin, tefluthrin, halfenprox, transfluthrin, bioresmethrin, pyrethrum extract, and more especially among prallethrin, 1R-trans-phenothrin, tetramethrin, d-tetramethrin, pyrethrins, imiprothrin, pyrethrum extract (i.e. pyrethrins).

Of special relevance are insecticide and/or acaricide formulations in accordance with the above characterised in that the target insects and mites are from the group: Ceratitis capitata, Bactrocera cucurbitae, Bactrocera oleae, Bactrocera carambolae, Bactrocera passiflorae, Bactrocera dorsalis, Musca domestica, Culex pipiens, Culex quinquefasciatus, Culex tarsalis, Culex tritaeniorhynchus, Anopheles quadriannulatus, Anopheles gambiae, Anopheles minimus, Anopheles dirus, Anopheles merus, Anopheles quadriannulatus, Anopheles arabiensis, Anopheles stephensi, Aedes australis, Aedes aegypti, Aedes africanus, Aedes albopictus, Aedes australis, Aedes atlanticus, Aedes atropalpus, Aedes brelandi, Aedes camptorhynchus, Aedes cantator, Aedes cataphylla, Aedes cinereus, Aedes dupreei, Aedes epactius, Aedes fulvus, Aedes grossbecki, Aedes hensilli, Aedes hesperonotius, Aedes infirmatus, Aedes intrudens, Aedes japonicus, Aedes melanimon, Aedes mitchellae, Aedes notoscriptus, Aedes polynesiensis, Aedes sollicitans, Aedes squamiger, Aedes taeniorhynchus, Aedes vexans, Aedes zoosophus, Ochlerotatus vigilax, Nycteribia pedicularia, Nycteribia kolenatii, Ixodes scapularis, Ixodes pacificus, Ixodes ricinus, Ixodes persulcatus, Ixodes sinensis, Ixodes kashmiricus, Ixodes kazakstani, Ixodes minor, Ixodes muris, Ixodes affinis, Ixodes rubicundus, Ixodes holocyclus, Ixodes cavipalpus, Dermacentor variabilis, Dermacentor andersoni, Rhipicephalus sanguineus, Amblyomma americio, Amblyomma maculatum, Otobius megnini, Tetranychus urticae, Panonychus citri, Dermatophagoides farinae, Dermatophagoides pteronyssinus, Euroglyphus maynei, Simulium erythrocephalum, Periplaneta americana, Blattella germanica, Blatta orientalis, Supella longipalpa, Blaptica dubia, Blaberus craniifer, Gromphadorhina portentosa.

Therefore, the invention contemplates the use of the molecules according to any of the paragraphs of this section above for pharmaceutical, veterinary, agricultural, urban pest control as well as domestic purposes.

The non-exclusive object type compounds of the present invention are, in accordance with a known CID nomenclature, used in the National Library of Medicine of the National Center for Biotechnology Information (NIH, USA) and in https://pubchem.ncbi.nlm.nih.gov/, and their CAS name:
1
   CID: 441314
   (2R,3R,4R,5S)-1-(2-hydroxyethyl)-2-(hydroxymethyl)piperidine-3,4,5-triol
2
   CID: 51634
   (2R,3R,4R,5S)-1-butyl-2-(hydroxymethyl)piperidine-3,4,5-triol
3
   CID: 501640
   (2R,3R,4R,5S)-2-(hydroxymethyl)-1-nonylpiperidine-3,4,5-triol
4
   CID: 501391
   (2R,3S,4R,5S)-1-butyl-2-(hydroxymethyl)piperidine-3,4,5-triol
5
   CID: 9859470
   (2S,3R,4R,5S)-2-(hydroxymethyl)-1-pentylpiperidine-3,4,5-triol
6
   CID: 4381
   2-(hydroxymethyl)-1-methylpiperidine-3,4,5-triol
7
   CID: 92381
   (2R,3R,4R,5S)-2-(hydroxymethyl)-1-methylpiperidine-3,4,5-triol
8
   CID: 9818954
   (2R,3R,4R,5S)-1-dodecyl-2-(hydroxymethyl)piperidine-3,4,5-triol
9
   CID: 6603107
   (2R,3R,4R,5S)-1-butyl-2-(hydroxymethyl)piperidine-3,4,5-triol hydrochloride
10
   CID: 129374
   (2R,3R,4R,5S)-2-(hydroxymethyl)-1-pentylpiperidine-3,4,5-triol
11
   CID: 12766473
   (2R,3R,4R,5S)-1-hexyl-2-(hydroxymethyl)piperidine-3,4,5-triol
12
   CID: 9882040
   (2R,3R,4R,5S)-2-(hydroxymethyl)-1-octylpiperidine-3,4,5-triol
13
   CID: 6477160
   (2R,3R,5S,6R)-2,6-bis(hydroxymethyl)-1-methylpiperidine-3,4,5-triol
14
   CID:49779547
   (2R,3R,4R)-2-(hydroxymethyl)-1-propylpiperidine-3,4-diol
15
   CID:44346217
   (2R,3R,4R,5S)-2-(hydroxymethyl)-1-(5-trimethylsilylpentyl)piperidine-3,4,5-triol16
16
   CID: 25010362
   (2S,3R,4R,5S)-2-(hydroxymethyl)-1-nonylpiperidine-3,4,5-triol
17
   CID: 25010186
   (2S,3R,4R,5S)-1-heptyl-2-(hydroxymethyl)piperidine-3,4,5-triol
18
   CID: 23622616
   (2S,3R,4R,5S)-1-butyl-2-(hydroxymethyl)piperidine-3,4,5-triol
19
   CID: 15221167
   (2R,3R,4R,5S)-2-(hydroxymethyl)-1-(4-trimethylsilylbutyl)piperidine-3,4,5-triol
20
   CID: 15221166
   (2R,3R,4R,5S)-2-(hydroxymethyl)-1-(3-trimethylsilylpropyl)piperidine-3,4,5-triol
21
   CID: 15218073
   (2R,3R,4R,5S)-2-(hydroxymethyl)-1-(trimethylsilylmethyl)piperidine-3,4,5-triol
22
   CID: 9881659
   (2R,3R,4R,5S)-1-heptyl-2-(hydroxymethyl)piperidine-3,4,5-triol
23
   CID: 6475031
   (2R,3S,5R,6R)-1-butyl-2,6-bis(hydroxymethyl)piperidine-3,4,5-triol
24
   CID: 3272526
   2-(hydroxymethyl)-1-nonylpiperidine-3,4,5-triol
25
   CID: 51577
   1-(2-hydroxyethyl)-2-(hydroxymethyl)piperidine-3,4,5-triol
26
   CID: 118458171
   (2R,3R,4R)-1-heptyl-2-(hydroxymethyl)piperidine-3,4-diol
27
   CID: 118458166
   (2R,3R,4R)-2-(hydroxymethyl)-1-(2-methoxyethyl)piperidine-3,4-diol
28
   CID: 70683132
   (2S,3S,4R)-1-butyl-2-(hydroxymethyl)piperidine-3,4-diol
29
   CID: 60169980
   (2R,3R,4R,5S)-1-(6-heptan-4-yloxyhexyl)-2-(hydroxymethyl)piperidine-3,4,5-triol
30
   CID: 53325719
   (2R,3R,4R,5S)-1-(5-butoxypentyl)-2-(hydroxymethyl)piperidine-3,4,5-triol
31
   CID: 53323483
   (2S,3R,4R,5S)-1-hexyl-2-(hydroxymethyl)piperidine-3,4,5-triol
32
   CID: 53320433
   (2S,3R,4R,5S)-1-(5-butoxypentyl)-2-(hydroxymethyl)piperidine-3,4,5-triol
33
   CID: 53320432
   (2S,3R,4R,5S)-2-(hydroxymethyl)-1-octylpiperidine-3,4,5-triol
34
   CID:44560309
   (2S,3R,4S,5S,6R)-1-butyl-6-(hydroxymethyl)piperidine-2,3,4,5-tetrol
35
   CID: 19826053
   2-(hydroxymethyl)-1-pentylpiperidine-3,4,5-triol
36
   CID: 16727511
   (2R,3R,4R,5S)-2-(hydroxymethyl)-1-(4,4,4-trifluorobutyl)piperidine-3,4,5-triol
37
   CID: 15605662
   (2R,3R,4R,5S)-1-(cyclopropylmethyl)-2-(hydroxymethyl)piperidine-3,4,5-triol
38
   CID: 15389573
   (2R,3R,4R,5S)-1-[[dimethyl(propyl)silyl]methyl]-2-(hydroxymethyl)piperidine-3,4,5-triol
39
   CID: 14849430
   2-(hydroxymethyl)-1-propylpiperidine-3,4,5-triol
40
   CID: 12766488
   2-(hydroxymethyl)-1-octylpiperidine-3,4,5-triol
41
   CID: 12766471
   1-hexyl-2-(hydroxymethyl)piperidine-3,4,5-triol
42
   CID: 11816841
   (2S,3S,4R,5S)-1-butyl-2-(hydroxymethyl)piperidine-3,4,5-triol
43
   CID: 6710690
   (4R,5R)-1-(2-hydroxyethyl)-2-(hydroxymethyl)piperidine-3,4,5-triol
44
   CID: 451512
   (2R,3R,4R,5S)-1-ethyl-2-(hydroxymethyl)piperidine-3,4,5-triol
45
   CID: 389416
   1-butyl-2,6-bis(hydroxymethyl)piperidine-3,4,5-triol
46
   CID: 128504
   (2R,3R,4R,5S)-2-(hydroxymethyl)-1,1-dimethylpiperidin-1-ium-3,4,5-triol
47
   CID: 76325709
   (2R,3R,4R,5S)-1-hexyl-2-(hydroxymethyl)piperidine-3,4,5-triol hydrochloride
48
   CID: 72204017
   (2R,3R,4R,5S,6R)-2-(hydroxymethyl)-1,6-dimethylpiperidine-3,4,5-triol
49
   CID: 70683133
   (2S,3R,4R)-1-butyl-2-(hydroxymethyl)piperidine-3,4-diol
50
   CID: 60170160
   (2R,3R,4R,5S)-1-(6-cyclopentyloxyhexyl)-2-(hydroxymethyl)piperidine-3,4,5-triol
51
   CID: 52944668
   (2S,3R,4S,5R,6R)-7-[(2R,3R,4R,5S)-3,4,5-trihydroxy-2-(hydroxymethyl)piperidin-1-yl]heptane-1,2,3,4,5,6-hexol;hydrochloride
52
   CID: 16727512
   (2R,3R,4R,5S)-2-(hydroxymethyl)-1-(5,5,5-trifluoropentyl)piperidine-3,4,5-triol
53
   CID:452002
   (2S,3S,4S,5S)-2-(hydroxymethyl)-1-methylpiperidine-3,4,5-triol
54
   CID: 129681909
   (3R,4S,5R,6R)-1-(1-hydroxyethyl)-6-(hydroxymethyl)piperidine-2,3,4,5-tetrol
55
   CID: 129669139
   (3R,4S,5R,6R)-1-butyl-6-(hydroxymethyl)piperidine-2,3,4,5-tetrol
56
   CID: 60169978
   (2R,3R,4R,5S)-2-(hydroxymethyl)-1-(6-pentane-3-yl-oxyhexyl)piperidine-3,4,5-triol
57
   CID:45483014
   (2R,3R,4S,5R)-5-fluoro-1-(2-hydroxyethyl)-2-(hydroxymethyl)piperidine-3,4-diol
58
   CID:40473182
   (2R,3R,4R,5S)-1-(2-hydroxyethyl)-2-(hydroxymethyl)piperidin-1-ium-3,4,5-triol
59
   CID: 9823155
   (2R,3R,4R,5S)-2-(hydroxymethyl)-1-octadecylpiperidine-3,4,5-triol
60
   CID:475539
   (2R,3R,4R,5S)-1-(2-heptoxyethyl)-2-(hydroxymethyl)piperidine-3,4,5-triol
61
   CID: 156509531
   (3R,4S,5S)-6-(4-aminopiperidin-1-yl)hexane-1,3,4,5-tetrol
62
   CID: 153904764
   (2R,3S,4R,5S)-1-(6-aminohexyl)-2-(hydroxymethyl)piperidine-3,4,5-triol
63
   CID: 146037504
   (2R,3R,4R,5R)-6-piperidin-1-ylhexane-1,2,3,4,5-pentol
64
   CID: 145759302
   (2R,3R,4R,5S)-1-butyl-2-(hydroxymethyl)piperidine-3,4,5-triol dihydrochloride
65
   CID: 142016736
   (3R)-1-(1,3-dihydroxypropan-2-yl)-2-(hydroxymethyl)piperidine-3,4-diol

On the other hand, other compounds also described with alpha-glucosidase or alpha-amylase activity derived from sugars, of the amino cyclitol glycoside type, also object of the present invention are:
66
   CID:444254
   (2*R*,3*R*,4*R*,5*S*,6*R*)-5-[(2*R*,3*R*,4*R*,5*S*,6*R*)-5-[(2*R*,3*R*,4*S*,5*S*,6*R*)-3,4-dihydroxy-6-methyl-5-[[(1S,4R,5S,6S)-4,5,6-trihydroxy-3-(hydroxymethyl)cyclohex-2-en-1-yl]amino]oxan-2-yl]oxy-3,4-dihydroxy-6- (hydroxymethyl)oxan-2-yl]oxy-6-(hydroxymethyl)oxane-2,3,4-triol
67
   CID:41774
   (3R,4R,5S,6R)-5-[(2R,3R,4R,5S,6R)-5-[(2R,3R,4S,5S,6R)-3,4-dihydroxy-6-methyl-5-[[(1S,4R,5S,6S)-4,5,6-trihydroxy-3-(hydroxymethyl)cyclohex-2-en-1-yl]amino]oxan-2-yl]oxy-3,4-dihydroxy-6-(hydroxymethyl)oxan-2-yl]oxy-6-(hydroxymethyl)oxane-2,3,4-triol
68
   CID: 129010024
   (2R,3R,4R,5S,6S)-5-[(2R,3R,4R,5S,6S)-5-[(2R,3R,4S,5S,6S)-3,4-dihydroxy-6-methyl-5-[[(1S,4R,5S,6R)-4,5,6-trihydroxy-3-(hydroxymethyl)cyclohex-2-en-1-yl]amino]oxan-2-yl]oxy-3,4-dihydroxy-6-(hydroxymethyl)oxan-2-yl]oxy-6-(hydroxymethyl)oxane-2,3,4-triol
69
   CID: 101101475
   (3R,4S,5S,6R)-6-[[(2S,3R,4R,5S,6R)-5-[(2R,3R,4S,5S,6R)-3,4-dihydroxy-6-methyl-5-[[(1S,4R,5S,6S)-4,5,6-trihydroxy-3-(hydroxymethyl)cyclohex-2-en-1-yl]amino]oxan-2-yl]oxy-3,4-dihydroxy-6-(hydroxymethyl)oxan-2-yl]oxymethyl]oxane-2,3,4,5-tetrol
70
   CID: 70684192
   (1S,2S,3R,6S)-6-[[(2S,3S,4S,5R,6S)-4,5-dihydroxy-2-(hydroxymethyl)-6-methoxyoxan-3-yl]amino]-4-(hydroxymethyl)cyclohex-4-ene-1,2,3-triol
71
   CID: 70682078
   (1 S,2S,3R,6S)-6-[[(2R,3S,4S,5R,6S)-4,5-dihydroxy-6-methoxy-2-methyloxan-3-yl]amino]-4-(hydroxymethyl)cyclohex-4-ene-1,2,3-triol
72
   CID: 197426
   (1S,2S,3R,6R)-6-[[(2R,3S,4S,5R,6S)-4,5-dihydroxy-6-methoxy-2-methyloxan-3-yl]amino]-4-(hydroxymethyl)cyclohex-4-ene-1,2,3-triol
73
   CID: 127767
   (1S,2S,3R,6R)-6-[[(2S,3S,4S,5R,6S)-4,5-dihydroxy-2-(hydroxymethyl)-6-methoxyoxan-3-yl]amino]-4-(hydroxymethyl)cyclohex-4-ene-1,2,3-triol
74
   CID: 101998608
   [(3S,4S,5S,6R)-3-[[(2R,3S,4S,5R,6R)-6-[(2R,3S,4R,5R,6R)-4,5-dihydroxy-2-(hydroxymethyl)-6-[(2R,3S,4R,5R)-4,5,6-trihydroxy-2-(hydroxymethyl)oxan-3-yl]oxyoxan-3-yl]oxy-4,5-dihydroxy-2-methyloxan-3-yl]amino]-4,5,6-trihydroxycyclohexen-1-yl]methyl hydrogen phosphate
75
   CID:444020
   (1S,2S,3R,4S,5S)-5-((1,3-dihydroxypropan-2-yl)amino)-1-(hydroxymethyl)cyclohexane-1,2,3,4-tetraol

We do not know the biochemical mechanism whereby the compounds of the present invention act as insecticides and their synergistic effect when combined with insecticides or acaricides is also unknown, apparently with greater activity if the insecticide or acaricide acts on sodium channels. While it is plausible that its insecticidal and/or synergistic activity is related to its alpha-glucosidase and/or alpha-amylase activity, it does not necessarily have to be so. Nor does the apparent biocomputational molecular fit of this type of molecules claimed in acetylcholinesterase guarantee that this is the mode of action. In short, the molecular basis underlying the insecticidal and/or synergistic activity of the compounds described is unknown, but it is plausible that it is related to their alpha-glucosidase inhibitory activity, even if that relationship is not cause-effect, and the structure that those compounds require to be alpha-glucosidase inhibitors, for some reason, coincides with the activity described in the present invention.

Furthermore, we have found that the possible theoretical activity of these molecules could be at the acetylcholinesterase level, but we lack experimental evidence beyond in silico assays.

In any case, the insecticidal and/or synergistic activity is evident in the compounds tested and reasonably expected in molecularly similar compounds.

The methodology for evaluating the insecticidal and/or synergistic activity of the compounds of the present invention is as follows:
The cockroaches are anaesthetised in their breeding box to pass them to 700mL containers for later selection by sex in zip bags. Each cockroach is taken out of the zip bag with the tweezers, gripping the individual by the chest at the height of the second pair of legs, and inserted into a zip bag.

The area chosen for the application of the active substances is in the ventral area of the thorax, where we can see more quickly if the substance has biocidal activity. For the application of the active substances, a 2-20µL manual pipette is used, which is adjusted to dose 5µL of the substance to be tested to each individual. The pipette tip is discarded between treatments. Once the substance has been applied to the ventral area of the thorax, the cockroach is immediately placed in the tub and the lid is closed quickly, but being careful not to catch the antennae. With a stopwatch we count the knockdown time. In the container, the sample number, treatment, replica and sex of the individual are noted. Data collection is carried out by noting the initial knockdown, that is, the first time the cockroach flips over and remains on its back for 3 seconds or more. It may recover, get back on its feet and walk again or move around the tub and then be knocked down again. Write down all the knockdowns successive to the initial knockdown that meet the previous description until the final knockdown. This parameter will be taken to make the survival graphs in Minitab. Some substances will not produce that "literal knockdown", in that case, the exact time in which the cockroach is paralysed, as without having force, should be noted. The affected parameter refers to the individual who is not able to move with coordination but is not dead (Guidance on the BPR: Volume II Parts B+C_Version 3.0 April 2018_pg. 334). In reality, it is difficult to assert when the cockroach is dead in an assay, since they can last for days with some sporadic leg movement without there being any possibility of recovery or any biological activity.

A similar process is performed for the application of the test products on flies, applying 0.2µL on the dorsal thorax, while holding the fly by a wing with the fingers without damaging the wing or the fly; they are then left in a tub with cotton soaked in 10% sucrose water.

The mosquito test is performed using cones as described in the WHO guidelines:
The WHO standard plastic cone is placed on a piece of filter paper attached with masking tape.

Each set of cone + filter paper will be dosed with a volume of 30µL of the corresponding formula on the paper with a micropipette.

From a mesh that contains only female mosquitoes (*Aedes albopictus*), about 20 individuals are collected with the entomological aspirator and placed into the cones.

The cones are then placed on a plastic surface held in forceps and held in a horizontal plane.

The knocked down mosquitoes are counted every 5 minutes up to 60 min. They are then collected and placed in a tub with cotton (water + 10% sucrose) to observe mortality at 24 hours.

From the tests carried out (all in adult insects), we can affirm that a dose of 50 grams of alpha-glucosidase inhibitor compound per kilogram of adult insect, causes a knockdown 50 (time in which the knockdown or paralysis of the insect occurs in 50% of the individuals tested) within 24 hours. Although it is a high dose, and difficult to formulate -except if they are considered suspended concentrates, with crystals dispersed in a continuous phase-, it is indicative of the insecticide and acaricide power *per se* of these compounds, far beyond the influence that is described as affecting larval development or molting (in the manner of insect growth regulators); that is, death or paralysis in a short space of time, which is necessary for the control of pests in adults on many occasions, is not previously described to our knowledge.

Although a high dose of alpha-glucosidase may be necessary for the control of insects and mites, nothing prevents it from being used in case of necessity, in particular for the use of polar solvents such as water, where the solubility of part of the compounds described is relatively high.

However, the greatest effect observed is undoubtedly in the synergy with insecticides involved in nerve transmission, and more particularly, in those insecticides that affect the sodium channels, and, in particular, and very notably, in pyrethrins and pyrethroids.

The invention includes the object of formulating the alpha-glucosidase inhibitors with other insecticides, so that said joint formulation is stable over time, i.e. in accordance with the requirements of the WHO with reference to pesticides and/or biocides, as well as being applicable for all forms of formulation available in pharmacy, medicine, veterinary, agriculture and pest control. To this end, the continuous phase can be both aqueous and oily, with the use of suitable surfactants to allow, in a manner known to the person skilled in the art, emulsions or microemulsions.

### Example 1. Synergistic effect of alpha-glucase inhibitors with pyrethroid insecticide (Prallethrin) in Periplaneta americana.

The following solutions are made in dimethylformamide (DMF) or dimethyl sulfoxide (DMSO), all the percentages mentioned in this document being in weight/weight, according to the following table, and 5µL are applied as explained above, in two *Periplaneta americana* individuals of each sex and per sample (n=4):

| Sample Code | Alpha-glucosidase inhibitor Pubchem Identifier | wt.-% | Biocide | wt.-% | Solvent | wt.-% |
|---|---|---|---|---|---|---|
| M1 | CID 441314 | 3 | Prallethrin | 0.05 | DMF | 96.95 |
| M2 | CID 444254 | 3 | Prallethrin | 0.05 | DMF | 96.95 |
| M2&1_Control | - | 0 | Prallethrin | 0.05 | DMF | 99.95 |
| M3 | CID 501640 | 3 | Prallethrin | 0.05 | DMSO | 96.95 |
| M4 | CID 501391 | 3 | Prallethrin | 0.05 | DMSO | 96.95 |
| M5 | CID 92381 | 3 | Prallethrin | 0.05 | DMSO | 96.95 |
| M6 | CID 9818954 | 3 | Prallethrin | 0.05 | DMSO | 96.95 |
| M7 | CID 444020 | 3 | Prallethrin | 0.05 | DMSO | 96.95 |
| M7-3_Control | - | 0 | Prallethrin | 0.05 | DMF | 99.95 |

From the results (in Graph 1) of Knockdown 50, the statistically significant synergistic effect is observed for all alpha-glucosidase inhibitors tested (from M1 to M7).

It is also remarkable that samples M1, M3, M4, M5 and M6 are also significantly and statistically different from samples M2 and M7, with M2 and M7 being alpha-glucosidase inhibitors but not of the iminosugar type and instead of the substituted tetrahydropyran type.

Mortality was also 100% in all treatments with alpha-glucosidase inhibitors, whereas in samples with Prallethrin alone, 100% mortality was not reached.

### Example 2. Synergistic effect of alpha-glucase inhibitors with pyrethroid insecticide (Prallethrin) in Musca domestica.

We proceed similarly with individuals of Musca domestica (also n=4 as in Example 1, two males and two females).

Results are shown in Graphs 3 and 4.

A less pronounced synergistic effect than in Periplaneta americana is observed in the Knockdown 50, however, the synergistic effect is more pronounced than in Periplaneta americana for the case of 24-hour mortality.

### Example 3. Synergistic effect of alpha-glucosidase inhibitors with pyrethroid insecticide (Prallethrin) in Aedes albopictus.

In this case, samples M8 (whose composition is exactly the same as M1) and M9 (of the same composition as M2) are again prepared and applied to Aedes albopictus in the manner described above using the WHO methodology, while the control in this example is the aforementioned M2&1_Control.

The results, shown in Graphs 5 and 6, show a marked synergistic effect, in which Knockdown 50 time is strongly reduced in the case of M8, while mortality is also influenced especially in the case of M9 and, to a lesser extent, by M9.

### Example 4. Alternative embodiments of the invention.

In the following table there are examples of embodiments of application of synergistic alpha-glucosidase inhibitors with insecticides according to the present invention:

| Target Species | Alpha-glucosidase inhibitor Pubchem Identifier | wt.-% | Coformulant | wt.-% | Solvent (and surfactant) | wt.-% | Amount applied (in µL) |
|---|---|---|---|---|---|---|---|
| Ixodes ricinus | CID: 441314 | 0.1 | Pyrethrins | 0.05 | Ethanol | 99.85 | 0.01 (capillary) |
| Ceratitis capitata | CID: 51634 | 1 | Chlorpyrifosmethyl | 1 | Acetone | 98 | 5 |
| Lasius niger | CID: 501640 | 1 | Sucrose | 10 | Water | 89 | ad libitum in cotton wick |
| Aedes aegypti | CID: 501391 | 3 | Metofluthrin | 0.05 | DMF | 96.95 | applied according to WHO TEST |
| Bemisia tabaci | CID: 9859470 | 0.1 | Abamectin | 0.01 | Water | 99.89 | ad libitum in cotton wick |
| Locusta migratoria | CID: 4381 | 10 | Deltamethrin | 0.1 | Tween 80 10% in Water | 89.9 | 50 |
| Musca domestica | CID: 92381 | 25 | Transfluthrin | 0.05 | Tween 80 10% in Water | 74.95 | 0.2 |
| Blatta orientalis | CID: 9818954 | 3 | Imiprothrin | 0.05 | Span 20 5% in D 20/26 (Cepsa, Spain) | 96.95 | applied according to WHO TEST |
| Anopheles gambiae | CID: 6603107 | 50 | Imidacloprid | 0.03 | Tween 20 3% in Water | 49.97 | applied according to WHO TEST |
| Culex pipiens | CID: 129374 | 6 | Prallethrin | 0.01 | DMSO | 93.99 | applied according to WHO TEST |
| Culex quinquefasc iatus | CID: 12766473 | 2 | D-Tetramethrin | 0.01 | DMF | 97.99 | applied according to WHO TEST |
| Blatta | CID: 444254 | 6 | Cyphenothrin | 0.1 | DMF 5% in | 93.9 | 5 |
| orientalis | | | | | D 20/26 | | |
| Periplaneta americana | CID:41774 | 2 | Aldrin | 0.01 | DMF | 97.99 | 0.01 (capillary) |
| Ceratitis capitata | CID: 101101475 | 1.5 | Carbofuran | 0.01 | DMF | 98.49 | 0.2 |
| Bactrocera cucurbitae | CID: 70684192 | 1 | Malathion | 0.01 | DMF | 98.99 | 0.2 |
| Aedes albopictus: | CID: 70682078 | 2 | Pyrethrins | 0.75 | DMSO | 97.25 | applied according to WHO TEST |
| Blattella germanica | CID: 197426 | 3 | Permethrin | 1 | DMSO | 96 | 5 |

## Claims

1. BIODEGRADABLE IMINOSUGARS AND AMINO CYCLITOL GLYCOSIDES INHIBITING ALPHA-GLUCOSIDASE WITH SYNERGISTIC INSECTICIDE OR ACARICIDE ACTIVITY OR BOTH as part of a compound comprising an insecticide, an acaricide or both as an active ingredient, with which the alpha-glucosidase inhibitor molecule is synergistic.

2. BIODEGRADABLE IMINOSUGARS AND AMINO CYCLITOL GLYCOSIDES INHIBITING ALPHA-GLUCOSIDASE WITH SYNERGISTIC INSECTICIDE OR ACARICIDE ACTIVITY OR BOTH according to claim 1, **characterised in that** their structure corresponds to structure (I) or (II): 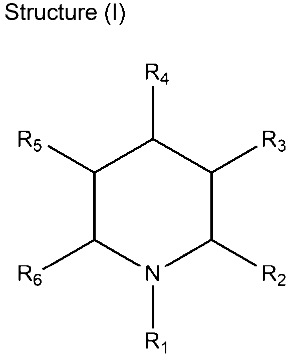 wherein,
R1 is chosen from: methyl, ethyl, propyl, butyl, pentyl, hexyl, heptyl, octyl, nonyl, decyl, undecanyl, dodecyl, tridecyl, tetradecyl, pentadecyl, hexadecyl, septadecyl, octadecyl; all of them alkanes comprising the linear chains and their positional isomers; hydroxymethyl, hydroxyethyl, hydroxypropyl, hydroxybutyl, hydroxypentyl, hydroxyhexyl, hydroxyheptyl, hydroxyoctyl, hydroxynonyl, hydroxydecyl, hydroxyundecanyl, hydroxydodecyl, hydroxytridecyl, hydroxytetradecyl, hydroxypentadecyl, hydroxyhexadecyl, hydroxyseptadecyl, hydroxyoctadecyl; all of them hydroxylated alkanes comprising the linear chains and their positional isomers which are hydroxylated, with the alcohol group being primary or secondary; heptan-4-yloxyhexyl, 5-butoxypentyl, 4,4,4-trifluorobutyl, 6-cyclohexyloxyhexyl, cyclopropylmethyl, dimethylpropylsilyl, cyclopentyloxyhexyl,
trimethylsilanylmethyl, trimethylsilanylethyl, trimethylsilanylpropyl, trimethylsinanylbutyl, trimethylsilylpentyl, as well as the particular cases where R1 is dimethyl, diethyl, dipropyl, i.e. two alkyls bound to the nitrogen;
R2, R3, R4, R5 and R6, independently of each other are chosen from: hydrogen, hydroxyl, hydroxymethyl, hydroxyethyl, hydroxypropyl, hydroxybutyl;
so that molecule (I) is a diol, triol or tetrol, with the exception of hydroxyls that may exist in R1;
as well as their possible salts, including those of hydrochlorides, including that of 3,4,5-trihydroxy-2-(hydroxymethyl)piperidin-1-yl)heptane-1,2,3,4,5,6-hexol. 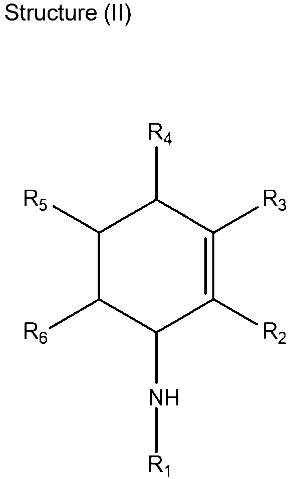
Wherein
R1 is chosen from: monosaccharide, disaccharide, trisaccharide, including isomaltotriose;
R2, R3, R4, R5 and R6, independently of each other are chosen from: hydrogen, hydroxyl, hydroxymethyl, hydroxyethyl, hydroxypropyl, hydroxybutyl;
or (II) is 5-(1,3-dihydroxypropan-2-ylamino)-1-(hydroxymethyl)cyclohexane-1,2,3,4-tetrol; with (I) and (II) comprising any possible stereoisomeric form of the depicted structures.

3. BIODEGRADABLE IMINOSUGARS AND AMINO CYCLITOL GLYCOSIDES INHIBITING ALPHA-GLUCOSIDASE WITH SYNERGISTIC INSECTICIDE OR ACARICIDE ACTIVITY OR BOTH according to any of the preceding claims, **characterised in that** it further comprises a biocide with which it acts synergistically.

4. BIODEGRADABLE IMINOSUGARS AND AMINO CYCLITOL GLYCOSIDES INHIBITING ALPHA-GLUCOSIDASE WITH SYNERGISTIC INSECTICIDE OR ACARICIDE ACTIVITY OR BOTH according to claim 3, **characterised in that** the biocide added is a pyrethroid or a pyrethrin.

5. BIODEGRADABLE IMINOSUGARS AND AMINO CYCLITOL GLYCOSIDES INHIBITING ALPHA-GLUCOSIDASE WITH SYNERGISTIC INSECTICIDE OR ACARICIDE ACTIVITY OR BOTH according to claim 4, **characterised in that** the added biocide is selected from prallethrin, 1R-trans-phenothrin, deltamethrin, tetramethrin, d-tetramethrin, methofluthrin, momfluorothrin, allethrin, cypermethrin, alpha-cypermethrin, beta-cypermethrin, zeta-cypermethrin, imiprothrin, lambda-cyhalothrin, gamma-cyhalothrin, bifenthrin, cyfluthrin, cyphenothrin, fenvalerate, fluvalinate, flumethrin, tralomethrin, resmethrin, kadethrin, permethrin, cis-permethrin, trans-permethrin, etofenprox, acrinathrin, flucythrinate, fenpropathrin, tefluthrin, halfenprox, transfluthrin, bioresmethrin, pyrethrum extract.

6. BIODEGRADABLE IMINOSUGARS AND AMINO CYCLITOL GLYCOSIDES INHIBITING ALPHA-GLUCOSIDASE WITH SYNERGISTIC INSECTICIDE OR ACARICIDE ACTIVITY OR BOTH according to any of the preceding claims, **characterised in that** the formulation is allowed to act on the insects and mites of the group: Ceratitis capitata, Bactrocera cucurbitae, Bactrocera oleae, Bactrocera carambolae, Bactrocera passiflorae, Bactrocera dorsalis, Musca domestica, Culex pipiens, Culex quinquefasciatus, Culex tarsalis, Culex tritaeniorhynchus, Anopheles quadriannulatus, Anopheles gambiae, Anopheles minimus, Anopheles dirus, Anopheles merus, Anopheles quadriannulatus, Anopheles arabiensis, Anopheles stephensi, Aedes australis, Aedes aegypti, Aedes africanus, Aedes albopictus, Aedes australis, Aedes atlanticus, Aedes atropalpus, Aedes brelandi, Aedes camptorhynchus, Aedes cantator, Aedes cataphylla, Aedes cinereus, Aedes dupreei, Aedes epactius, Aedes fulvus, Aedes grossbecki, Aedes hensilli, Aedes hesperonotius, Aedes infirmatus, Aedes intrudens, Aedes japonicus, Aedes melanimon, Aedes mitchellae, Aedes notoscriptus, Aedes polynesiensis, Aedes sollicitans, Aedes squamiger, Aedes taeniorhynchus, Aedes vexans, Aedes zoosophus, Ochlerotatus vigilax, Nycteribia pedicularia, Nycteribia kolenatii, Ixodes scapularis, Ixodes pacificus, Ixodes ricinus, Ixodes persulcatus, Ixodes sinensis, Ixodes kashmiricus, Ixodes kazakstani, Ixodes minor, Ixodes muris, Ixodes affinis, Ixodes rubicundus, Ixodes holocyclus, Ixodes cavipalpus, Dermacentor variabilis, Dermacentor andersoni, Rhipicephalus sanguineus, Amblyomma americio, Amblyomma maculatum, Otobius megnini, Tetranychus urticae, Panonychus citri, Dermatophagoides farinae, Dermatophagoides pteronyssinus, Euroglyphus maynei, Simulium erythrocephalum, Periplaneta americana, Blattella germanica, Blatta orientalis, Supella longipalpa, Blaptica dubia, Blaberus craniifer, Gromphadorhina portentosa.

7. The use of the molecules according to any of the preceding claims for pharmaceutical, veterinary, agricultural, urban pest control, as well as domestic, purposes.
